# EUROPÄISCHE PATENTSCHRIFT

(11) **EP 1 406 887 B1**
(45) Veröffentlichungstag und Bekanntmachung des Hinweises auf die Patenterteilung: **19.01.2005**
(21) Anmeldenummer: 02758298.0
(22) Anmeldetag: 03.07.2002
(51) Int. Cl.: C07D 307/08, C07D 315/00, B01J 23/89

(54) **VERFAHREN ZUR SELEKTIVEN HERSTELLUNG VON TETRAHYDROFURAN DURCH HYDRIERUNG VON MALEINSÄUREANHYDRID**
METHOD FOR THE SELECTIVE PRODUCTION OF TETRAHYDROFURAN BY HYDROGENATING MALEIC ACID ANHYDRIDE
PROCEDE DE PRODUCTION SELECTIVE DE TETRAHYDROFURANNE PAR HYDROGENATION D'ANHYDRE DE L'ACIDE MALEIQUE

(30) Priorität: 07.07.2001 DE 10133054
(43) Veröffentlichungstag der Anmeldung: 14.04.2004
(73) Patentinhaber: BASF AKTIENGESELLSCHAFT, 67056 Ludwigshafen (DE)
(72) Erfinder: SCHLITTER, Stephan, 67117 Limburgerhof (DE); BORCHERT, Holger, 67591 Offstein (DE); HESSE, Michael, 67549 Worms (DE); SCHUBERT, Markus, 67063 Ludwigshafen (DE); BOTTKE, Nils, 68165 Mannheim (DE); FISCHER, Rolf-Hartmuth, 69121 Heidelberg (DE); RÖSCH, Markus, 55276 Dienheim (DE); HEYDRICH, Gunnar, 67117 Limburgerhof (DE); WECK, Alexander, 67251 Freinsheim (DE)
(86) Internationale Anmeldenummer: PCT/EP2002/007341
(87) Internationale Veröffentlichungsnummer: WO 2003/006445

(56) Entgegenhaltungen:
- US-A- 1 036 510
- US-A- 3 224 981
- US-A- 4 083 809
- US-A- 5 072 009
- Periodensystem der Elemente, VCH-Verlagsgesellschaft, Weinheim, 1995, Best.-Nr. 1010120. XP002214647

## Beschreibung

Die vorliegende Erfindung betrifft ein Verfahren zur Herstellung von gegebenenfalls alkylsubstituiertem γ-Butyrolacton und Tetrahydrofuran durch katalytische Hydrierung in der Gasphase von Substraten, die ausgewählt sind aus der Gruppe bestehend aus Maleinsäure und Bernsteinsäure und Derivaten dieser Säuren. Darunter werden im Rahmen der vorliegenden Erfindung Ester und Anhydride verstanden, wobei diese ebenso wie die Säuren einen oder mehrere Alkylsubstituenten aufweisen können. Es wird ein Vollkatalysator verwendet, der aus Kupferoxid, Palladium und/oder einer Palladiumverbindung sowie mindestens einem weiteren Metalloxid besteht.

Die Herstellung von γ-Butyrolacton (GBL) und Tetrahydrofuran (THF) durch Gasphasenhydrierung von Maleinsäureanhydrid (MSA) ist eine seit vielen Jahren bekannte Reaktion. Zur Durchführung dieser katalytischen Reaktion sind in der Literatur zahlreiche Katalysatorsysteme beschrieben. Diese sind zum großen Teil Crhaltig. Je nach Zusammensetzung des Katalysators und den gewählten Reaktionsparametern werden mit derartigen Katalysatoren unterschiedliche Produktverteilungen erreicht.

Mögliche weitere Edukte zur Herstellung von GBL und THF sind neben MSA die Maleinsäure selbst, Bernsteinsäure und deren Anhydrid sowie die Ester dieser Säuren. Sollen GBL und THF hergestellt werden, die Alkylsubstituenten aufweisen, so bietet es sich an, von den vorstehend genannten Säuren, Estern und Anhydriden auch die entsprechend alkylsubstituierten Spezies zu verwenden.

In der US 3,065,243 ist ein Verfahren offenbart, bei dem Kupferchromit als Katalysator dient. Laut Beschreibung und Beispielen entstehen bei dieser Reaktionsführung noch beträchtliche Mengen an Bernsteinsäureanhydrid (BSA), das im Kreis gefahren werden muß. Wie bekannt ist, treten dabei häufig verfahrenstechnische Probleme aufgrund der Kristallisation des BSA oder auch daraus entstehender Bernsteinsäure mit anschließender Verstopfung von Rohrleitungen auf.

Die Offenbarung von weiteren Kupferchromit-Katalysatoren zur Hydrierung von MSA findet sich zum Beispiel in den Druckschriften US 3,580,930, US 4,006,165, der EP-A 638 565 sowie der WO 99/38856. Laut Offenbarung lassen sich mit den dort beschriebenen Katalysatoren hohe Ausbeuten an GBL erzielen. THF wird jeweils nur in Spuren gebildet. Oftmals ist es jedoch so, daß höhere Mengen an THF aus mehreren Gründen erwünscht sind.

Ein Verfahren, das dies gestattet, wird in der US 5,072,009 offenbart. Die gemäß diesem Patent verwendeten Katalysatoren entsprechen der allgemeinen Formel Cu1ZnbA1cMdox, in der M mindestens ein Element ist, das ausgewählt ist aus der Gruppe bestehend aus den Gruppen IIA und IIIA, VA, VIII, Ag, Au, den Gruppen IIIB bis VIIB sowie Lanthaniden und Aktinoiden des Periodensystems der Elemente; b ist eine Zahl zwischen 0,001 und 500, c eine Zahl zwischen 0,001 und 500 und d eine Zahl von 0 bis < 200 und x entspricht der Anzahl an Sauerstoffatomen, die nach den Valenzkriterien notwendig sind. Obwohl ausgesagt wird, daß die Katalysatoren entsprechend dieser Patentschrift kein Chrom enthalten müssen, werden in allen Beispielen chromhaltige Katalysatoren beschrieben. Nach diesen Beispielen wird eine maximale THF-Ausbeute von 96% erhalten, die Hydrierung wird bei Drücken von 20 bis 40 bar durchgeführt.

Ein zweistufiges Katalysatorsystem zur Hydrierung von MSA ist in der Patentschrift US 5,149,836 beschrieben. Der Katalysator für die erste Stufe ist chromfrei, der Katalysator für die zweite Stufe basiert auf Cu-Zn-Cr-Oxiden.

Prinzipiell nachteilig an allen oben beschriebenen Katalysatorsystemen ist die Anwesenheit von Chromoxid, dessen Verwendung aufgrund der akuten Toxizität vermieden werden sollte. Auch derartige Cr-freie Katalysatorsysteme zur Herstellung von GBL durch Hydrierung von MSA sind im Stand der Technik beschrieben. Beispiele für derartige Katalysatorsysteme finden sich in den Druckschriften WO 99/35139 (Cu-Zn-Oxid), WO 95/22539 (Cu-Zn-Zr) sowie der US 5,122,495 (Cu-Zn-Al-Oxid). Alle diese Katalysatorsysteme ermöglichen hohe Ausbeuten an GBL, bis zu 98%, dabei wird die Bildung von THF jedoch nicht oder nur in Spuren beobachtet. Zwar läßt sich dessen Bildung, wie bekannt ist, durch eine Erhöhung der Reaktionstemperatur oder längere Verweilzeiten im Reaktor begünstigen, gleichzeitig steigt jedoch auch der Anteil unerwünschter Nebenprodukte, beispielsweise Butanol, Butan, Ethanol oder Ethan.

Ein ausschließlich aus Cu- und Al-Oxiden aufgebauter Katalysator für die MSA-Gasphasenhydrierung zu GBL wird in der WO 97/24346 offenbart. Auch hier finden sich die gleichen Nachteile wie bei den im vorstehenden Absatz beschriebenen Druckschriften, nämlich nur untergeordnete Bildung von THF, die lediglich durch extreme Reaktionsbedingungen gesteigert werden kann.

Die Verwendung eines Katalysators mit prinzipiell gleicher Zusammensetzung wie in der WO 97/24346 beschrieben, nämlich basierend auf Cu-Al-Oxiden, wird auch in der JP 2 233 631 offenbart. Das Ziel dieser Erfindung liegt dabei darin, die Hydrierung von MSA so durchzuführen, dass als Hauptprodukte THF und 1,4-Butandiol neben nur geringen oder gar keinen Mengen GBL entstehen. Dieses wird dann durch die Verwendung der auf gemischten Cu-Al-Oxiden basierenden Katalysatoren sowie durch Einhalten bestimmter Reaktionsbedingungen erreicht. Typische, mit diesem Verfahren erhaltene Mischungen enthalten ca. 15 bis 20 Mol-% 1,4-Butandiol und 60 bis 80 Mol-% THF, wobei die Menge an THF sogar entsprechend einem Beispiel auf über 99 Mol-% gesteigert werden kann. Dies wird dadurch erreicht, daß GBL als Lösungsmittel eingesetzt wird, und zwar in einem mehrfachen Überschuß. Wird dagegen ohne Lösungsmittel gearbeitet, sinken die Ausbeuten beträchtlich, auf Werte um 75%.

In der US 4,105,674 wird ein Verfahren zur Hydrierung von Maleinsäure, Bernsteinsäure oder deren Anhydriden an gegebenenfalls geträgerten Cu-Pd oder Cu-Pt Katalysatoren offenbart. Ziel der Erfindung ist die Herstellung von GBL in hohen Ausbeuten und unter Bildung von möglichst wenig Nebenprodukten wie THF und Butanol. Zu diesem Zweck wird als Träger bevorzugt ein nichtacides Material, in den Bespielen durchweg Magnesiumsilikat, verwendet. Die erfindungsgemäßen Katalysatoren erreichen Selektivitäten zu GBL von über 90%; die Selektivität zu THF wird generell zu 2-10% angegeben.

Alle die in den vorstehend genannten Druckschriften beschriebenen Katalysatortypen weisen den Nachteil auf, dass sie noch eine große Menge an unerwünschtem Nebenprodukt liefern bzw. nur für die Herstellung von GBL befriedigende Aktivitäten erreicht werden. Häufig enthalten die Katalysatoren auch Cr.

Die Aufgabe der vorliegenden Erfindung besteht darin, einen Katalysator für die Gasphasenhydrierung von Maleinsäure und/oder Bernsteinsäure und/oder den oben erwähnten Derivaten zur Verfügung zu stellen, der hohe Selektivtäten zu gegebenenfalls substituiertem THF liefert. Dieser Katalysator soll unter entsprechenden Reaktionsbedingungen hohe Ausbeuten an THF mit gleichzeitig geringer Bildung von unerwünschtem Nebenprodukt ermöglichen. Der Katalysator soll dabei Cr-frei sein.

Diese Aufgabe wird gelöst durch einen Katalysator für die Hydrierung von C4-Dicarbonsäuren und/oder deren Derivaten in der Gasphase, dadurch gekennzeichnet, dass dieser Katalysator
a) 20-94 Gew.-% Kupferoxid (CuO), vorzugsweise 40-92 Gew.% CuO, insbesondere 60-90 Gew.-% CuO, und
b) 0,005-5 Gew.-%, vorzugsweise 0,01-3 Gew.-%, insbesondere 0,05-2 Gew.-% Palladium und/oder einer Palladiumverbindung (berechnet als metallisches Palladium) und
c) 2-79,995 Gew.-%, vorzugsweise 5-59,99 Gew.-%, insbesondere 8-39,95 Gew.-% eines oxidischen Trägers ausgewählt aus der Gruppe der Oxide des Al, Si, Zn, La, Ce, der Elemente der Gruppen IIIA bis VIIIA sowie der Gruppen IA und IIA, des Periodensystems der Elemente aufweist.

Die erfindungsgemäßen Katalysatoren erlauben es, die Hydrierung von C4-Dicarbonsäuren und/oder deren Derivaten in der Gasphase so durchzuführen, dass als Hauptprodukt ein gegebenenfalls alkylsubstituiertes Tetrahydrofuran entsteht, und zwar in Ausbeuten von deutlich über 90%. Überraschenderweise wurde gefunden, dass der Zusatz von Palladium als Aktivmetall einen deutlichen Einfluß auf die Selektivität zu THF hat.

Die Gruppen des Periodensystems der Elemente werden im Zusammenhang mit der vorliegenden Erfindung nach der alten IUPAC-Nomenklatur bezeichnet.

Unter dem Begriff C₄-Dicarbonsäuren und deren Derivate werden im Bezug auf die vorliegende Anmeldung Maleinsäure und Bernsteinsäure verstanden, die gegebenenfalls einen oder mehrere C1-C6-Alkylsubstituenten aufweisen sowie die Anhydride und Ester dieser gegebenenfalls alkylsubstituierten Säuren. Ein Beispiel einer solchen Säure ist Citraconsäure. Vorzugsweise wird MSA eingesetzt. Das hergestellte THF kann, je nach eingesetztem Ausgangsmaterial, dabei auch ein oder mehrere Alkylsubstituenten aufweisen.

Die erfindungsgemäßen Katalysatoren weisen an sich bekanntes Cu-Oxid, Palladium und/oder eine Palladiumverbindung; bevorzugt Palladiumoxid und/oder Palladiumnitrat, und einen oxidischen Träger mit sauren Zentren auf. Vorzugsweise enthalten die Katalysatoren kein Cr. Die Katalysatoren können als Formkörper verwendet werden, beispielsweise als Stränge, Rippstränge, andere Extrudatformen, Tabletten, Ringe, Kugeln und Splitt.

Geeignete Trägermaterialien sind eines oder mehrere der Oxide von Elementen aus der Gruppe bestehend aus Al, Si, Zn, La, Ce, den Elementen der Gruppen IIIA bis VIIIA sowie den Gruppen IA und IIA. Der Träger weist bevorzugt eine geeignete Anzahl saurer Zentren auf. Vorzugsweise wird ein Oxid der Elemente aus der Gruppe bestehend aus Al, Si, Ti, Zn, Zr und/oder Ce eingesetzt. Insbesondere geeignet ist Al. Der Träger wird in einer Menge von <80 Gew.-% bezogen auf den gesamten Katalysator eingesetzt. Die Menge an Kupferoxid liegt bei Werten von >20 Gew.-% und die Menge an Palladium und/oder Palladiumverbindung bei <5 Gew.-%.

Der erfindungsgemäße Katalysator besteht vorzugsweise ausschließlich aus Kupferoxid, Palladium und/oder einer Palladiumverbindung und Aluminiumoxid, neben den üblichen, dem Fachmann bekannten Verunreinigungen.

Die erfindungsgemäßen Katalysatoren können zudem Hilfsmittel in einer Menge von 0 bis 10 Gew.% enthalten. Unter Hilfsmittel versteht man organische und anorganische Stoffe, die zu einer verbesserten Verarbeitung während der Katalysatorherstellung und/oder zu einer Erhöhung der mechanischen Festigkeit der Katalysatorformkörper beitragen. Derartige Hilfsmittel sind dem Fachmann bekannt und umfassen Graphit, Stearinsäure, Kieselgel und Kupferpulver.

Die erfindungsgemäßen Katalysatoren werden nach dem Fachmann an sich bekannten Methoden beispielsweise durch gemeinsame Fällungen aller oder von mindestens zwei Komponenten (Cofällungen), Fällungen der einzelnen Komponenten mit nachfolgenden innigen Vermischen, zum Beispiel durch Kneten oder Kollern, Tränken des oxidischen Trägers mit den anderen Komponenten a) und b) in einem oder in mehreren Schritten hergestellt. Weiterhin können die erfindungsgemäßen Katalysatoren durch Verformen einer heterogenen Mischung aus den Komponenten a), b) und c) erhalten werden.

Bevorzugt sind Verfahren, bei denen das Kupferoxid fein verteilt und innig vermischt mit dem Trägermaterial anfällt, besonders bevorzugt sind Fällungsreaktionen. Der erfindungsgemäße Katalysator kann beispielsweise durch Ausfällen der entsprechenden Metallcarbonate und/oder Hydroxide in wässriger Lösung, Waschen, Trocknen und Calcinieren hergestellt werden. Die Metallcarbonate oder Hydroxide sind beispielsweise durch Lösen der entsprechenden Metallsalze in Wasser und Zugabe eines Fällungsmittels, bevorzugt Sodalösung, erhältlich. Als Metallsalze kommen beispielsweise, Nitrate, Sulfate, Chloride, Acetate oder Oxalate zum Einsatz. Nach dem Ausfällen wird der erhaltene Niederschlag abfiltriert, gewaschen, getrocknet und gegebenenfalls calciniert. Palladium und/oder die Palladiumverbindung kann dabei gleichzeitig mit den anderen Komponenten gefällt werden oder kann dem Fällungsprodukt auf jeder Verarbeitungsstufe zugesetzt werden.

Eine derart hergestellte Aktivmasse aus der Komponente a) oder aus den Komponenten a) und b) kann auf den Träger c) in üblicher Weise aufgebracht werden, beispielsweise durch Kollern oder Kneten, ggf. in Gegenwart eines Binders, Klebers oder Peptisierungsmittels. Es können auch andere bekannte Verfahren eingesetzt werden, um das Trägermaterial c) mit einer Aktivmasse aus den Komponenten a) und b) zu versetzen. Beispielsweise kann das Trägermaterial als Pulver oder Formkörper mit Precursor-Substanzen der Aktivmasse, beispielsweise den erwähnten Nitraten, Sulfaten, Chloriden, Acetaten oder Oxalaten oder Hydroxiden der jeweiligen Metalle oder entsprechenden Lösungen innig vermengt, beschichtet oder getränkt werden. Dieser vorbehandelte Träger wird dann zur Herstellung der Aktivmasse einer Wärmebehandlung unterzogen.

Besonders bevorzugt erfolgt die Katalysatorherstellung jedoch durch Cofällung der Komponenten a) und c) und die anschließende Tränkung der entsprechenden nach Trocknung erhaltenen Masse oder des nach dem Pressen dieser Masse erhaltenen Formkörpers mit einer wässrigen Lösung einer löslichen Palladiumverbindung, insbesondere einer Palladiumsalzlösung beispielsweise einer Lösung des Nitrats, Acetats, Acetylacetonats, Propionats, Palladiumtetraminacetats oder Palladium-tetraminnitrats, wobei Palladiumnitrat bevorzugt ist. Die mit der Palladiumsalzlösung getränkten Massen oder Formkörper werden anschließend vorzugsweise bei Temperaturen von 50 bis 150°C getrocknet und wahlweise bei Temperaturen von 150 bis 800°C calciniert. Die Tränkung eines Formkörpers ist bevorzugt.

Generell wird der Katalysator vor dem Einsatz in die Reaktion einer Aktivierung, im allgemeinen einer Wasserstoffvorbehandlung, unterzogen. Dadurch wird die aktive Katalysatorspezies hergestellt. Dies geschieht durch ein teilweises Reduzieren der in der Katalysatormischung vorhandenen Oxide zum elementaren Metall, das in der erfindungsgemäßen katalytischen Reaktion aktiv ist.

Der erfindungsgemäße Katalysator besitzt eine ausreichende Standzeit. Für den Fall, daß die Aktivität und/oder Selektivität des Katalysators dennoch im Laufe seiner Betriebszeit sinken sollte, kann er durch dem Fachmann bekannte Maßnahmen regeneriert werden. Hierzu zählt eine reduktive Behandlung des Katalysators im Wasserstoffstrom bei erhöhter Temperatur. Gegebenenfalls kann der reduktiven Behandlung eine oxidative vorausgehen. Hierbei wird die Katalysatorschüttung mit einem molekularen Sauerstoff enthaltenden Gasgemisch, beispielsweise Luft, bei erhöhter Temperatur durchströmt. Weiterhin besteht die Möglichkeit, den Katalysator mit einem geeigneten Lösungsmittel, beispielsweise Methanol, THF oder GBL, zu waschen und anschließend durch einen Gasstrom zu trocknen.

Für die Umsetzung der Reaktion sind Reaktoren, in denen der Katalysator als Festbett angeordnet ist, geeignet. Besonders bevorzugt sind Rohrbündelreaktoren, um die bei der Reaktion frei werdende Wärme günstig abzuführen. MSA wird verdampft und mit einem Wasserstoff enthaltenden Gasstrom durch den Reaktor geleitet. Bevorzugt ist ein Gemisch mit hohem Wasserstoffgehalt. In einigen Fällen wirkt sich die Zuführung anderer gasförmiger Komponenten, wie Wasserdampf, Kohlenwasserstoffe wie beispielsweise Methan, Ethan oder n-Butan oder Kohlenmonoxid, günstig auf die Selektivität, Aktivität oder Langzeitstabilität aus.

Die Konzentration des MSA liegt bei 0,1 bis 5 Vol.-%, bevorzugt zwischen 0,2 und 2 Vol.-%. Bei wesentlich höheren MSA-Konzentrationen kondensiert MSA im Reaktor aus und belegt den Katalysator mit einem Flüssigkeitsfilm. Wesentlich geringere Konzentrationen als die vorstehend angegebenen sind prinzipiell möglich, jedoch würden diese die Raum-Zeit-Ausbeute verringern und das Verfahren unnötig verteuern. Die Temperatur der Reaktion wird auf Werte von 150 bis 400°C, vorzugsweise 200 bis 300°C, eingestellt. Höhere Temperaturen begünstigen die Bildung von Nebenprodukten, tiefere Temperaturen führen zu einem unnötigen Aktivitätsverlust des Katalysators. Der Druck wird auf Werte von 0,5 bis 50 bar, vorzugsweise 1 bis 20 bar eingestellt. Die GHSV (Gas Hourly Space Velocity = Volumenstrom des Reaktionsgases bei Normalbedingungen bezogen auf das Katalysatorschüttvolumen) wird so eingestellt, daß ein vollständiger MSA-Umsatz erreicht wird. Dies erleichtert die Aufarbeitung des Produktgemisches und erspart die Rückführung von nicht umgesetztem MSA. Die GHSV liegt bei 10 bis 50 000 h⁻¹, vorzugsweise zwischen 100 und 10.000 h⁻¹. Das Produktgemisch kann nach dem Fachmann bekannten Verfahren getrennt werden. Bevorzugt wird mindestens ein Teil des nicht umgesetzten Wasserstoffs im Kreis gefahren und damit erneut in der Hydrierung eingesetzt.

Es hat sich gezeigt, daß durch Variation der Reaktionsparameter die Bildung der gewünschten Endprodukte gesteuert werden kann. Dies sind insbesondere Druck, Temperatur und GHSV. So läßt sich generell bei hohen Werten von Druck und Temperatur sowie niedrigen GHSV-Werten eine erhöhte, teils auch ausschließliche Bildung von THF feststellen. Dagegen führen niedrige Druck- und Temperaturwerte und hohe GHSV-Werte zur vermehrten Bildung von GBL.

Die Erfindung wird nun in den nachfolgenden Beispielen erläutert.

### Vergleichsbeispiel V 1:

### Herstellung von CuO/Al₂O₃-Katalysatortabletten

In einem beheizbaren und mit Rührwerk ausgestatteten Fälltopf werden 31 Wasser vorgelegt und auf 80°C erwärmt. In dieses Fällgefäß werden im Verlauf einer Stunde eine Metallsalzlösung bestehend aus 1754 g Cu(NO₃)₂*2, 5H₂O und 2944 g Al(NO₃)₃*9H₂O in 4000 ml Wasser und gleichzeitig eine 20 Gew.-% Sodalösung unter Rühren zudosiert. Die Sodadosierung wird so gewählt, dass sich im Fällgefäß ein pH-Wert von 6 einstellt. Nach vollständiger Zugabe der Metallsalzlösung wird weiter Sodalösung zudosiert, bis im Fällgefäß ein pH-Wert von 8 erreicht ist und weitere 15 min bei diesem pH-Wert gerührt. Der Gesamtverbrauch an Sodalösung liegt bei 11 kg. Die gebildete Suspension wird abfiltriert und mit Wasser gewaschen, bis das ablaufende Waschwasser kein Nitrat (<25 ppm) mehr enthält. Der Filterkuchen wird zunächst bei 120°C getrocknet und anschließend bei 600°C calciniert.

600 g dieses Materials werden mit 18 g Graphit intensiv vermischt und zu Tabletten mit Durchmesser und Höhe jeweils 3 mm tablettiert.

### Beispiel 2:

### Herstellung eines erfindungsgemäßen CuO/PdO/Al₂O₃-Katalysators

550 g der Tabletten aus Beispiel 1 mit einer Wasseraufnahme von 0,33 cm³/g wurden in einer Tränktrommel mit einer Lösung enthaltend 2,76 g Pd als Palladiumnitrat in 172 ml Wasser gleichmäßig besprüht, bei 100°C getrocknet und schließlich 2h bei 350°C calciniert.

### Vergleichsbeispiel V3:

### Herstellung eines CuO/PtO/Al2O3-Vergleichskatalysators:

550g der Tabletten aus Beispiel 1 mit einer Wasseraufnahme von 0,33 cm³/g wurden in einer Tränktrommel mit einer Lösung enthaltend 2,76 g Pt als Platinnitrat in 172 ml Wasser gleichmäßig besprüht, bei 100°C getrocknet und schließlich 2h bei 350°C calciniert.

### Vergleichsbeispiel V 4, Beispiel 5, Vergleichsbeispiel V 6:

### Hydrierung von Maleinsäureanhydrid

Jeweils 100 ml der tablettierten Katalysator-Formkörper gemäß Vergleichsbeispiel V 1 oder Beispiel 2 wurden mit 100 ml Glasringen der gleichen Größe gemischt und in einen Rohrreaktor mit 27 mm Innendurchmesser gefüllt. Der Reaktor wurde durch umlaufendes Öl temperiert und von oben nach unten mit dem Reaktorgas durchströmt. MSA wurde als Schmelze in einen bei 200°C betriebenen Verdampfer gepumpt, wo es in einem Wasserstrom verdampft wurde. Das MSA-Wasserstoff-Gemisch mit einer MSA-Konzentration von 1,2 Vol.-% wurde dann durch den Reaktor geleitet und oberhalb der Katalysatorschüttung vorgeheizt. Der Umsatz von MSA war in allen Beispielen vollständig.

Vor dem Einspeisen des MSA-Wasserstoffgemisches wurde der Katalysator einer Wasserstoffvorbehandlung unterzogen. Dazu wurde zunächst der Reaktor mit 200 Nl/h Stickstoff bei atmosphärischem Druck gespült und gleichzeitig innerhalb einer Stunde auf eine Temperatur in der Katalysatorschüttung von 180°C aufgeheizt. Danach wurde der Stickstoffstrom auf 950 hl/h erhöht und zusätzliche 50 Nl/h Wasserstoff eingespeist. Dabei wurde eine leichte Temperaturerhöhung in der Katalysatorschüttung auf etwa 250°C im Hot-Spot beobachtet. Der Hot-Spot wandert vom Reaktoreingang zum Reaktorende durch den Reaktor. Nachdem die Temperatur in der gesamten Katalysatorschüttung auf 190°C abgekühlt war, wurde der Stickstoffstrom auf 900 Nl/h erniedrigt und die Menge des Wasserstromes auf 100 Nl/h erhöht. Der Stickstoffvolumenstrom wurde allmählich abgeschaltet, der Wasserstoffstrom allmählich auf 250 Nl/h angehoben.

Um die Aktivität der Katalysatoren zu vergleichen wurde die GHSV von 2500 auf 6000 h⁻¹ erhöht.

| Bsp. Nr. | Kat. Nr. | T [°C] | GHSV [1/h] | S_{BSA} [mol-%] | S_{GBL} [mol-%] | S_{THF} [mol-%] | S_{Rest} [mol-%] | Raum-Zeit-Ausbeute [g_{THF}/ hl_{Kat.}] |
|---|---|---|---|---|---|---|---|---|
| V 4 | V 1 | 250 | 2500 | 0 | 0 | 93 | 7 | 89 |
| | | 250 | 3000 | < 1 | 13 | 83 | 7 | 95 |
| | | 250 | 6000 | 30 | 62 | 7 | 1 | 16 |
| 5 | 2 | 250 | 2500 | 0 | 0 | 89 | 11 | 85 |
| | | 250 | 3000 | 0 | 0 | 91 | 9 | 104 |
| | | 250 | 6000 | 0 | 0 | 93 | 7 | 212 |
| V 6 | V 3 | 250 | 2500 | 0 | 2 | 76 | 22 | 73 |
| | | 250 | 3000 | 10 | 71 | 15 | 4 | 17 |
| | | 250 | 6000 | 32 | 59 | 7 | 2 | 16 |
| Sₓₓₓ = Selektivität des jeweiligen Produkts | | | | | | | | |

Wie die Tabelle zeigt wird bei dem Pd freien Katalysator gemäß Vergleichsbeispiel 1 die maximale Raum-Zeit-Ausbeute zu Tetrahydrofuran bei einer GHSV von 3000 h⁻¹ erreicht. Die THF-Selektivität von 93 % erreicht der Katalysator bei einer Raum-Zeit-Ausbeute von 89 g _{THF}/hl_{Katalysator.}

Im Vergleich dazu wird mit dem erfindungsgemäßen Katalysator aus Beispiel 2 bei gleicher Tetrahydrofuranselektivität von 93 % eine Steigerung der Raum-Zeit-Ausbeute auf 212 g _{THF}/h*l_{Katalysator} erzielt. Der Platin-dotierte Katalysator aus Vergleichsbeispiel 3 zeigt hingegen eine verschlechterte Aktivität und Selektivität zu Tetrahydrofuran im Vergleich zum nicht dotierten System.

## Patentansprüche

1. Katalysator für die Hydrierung von C₄-Dicarbonsäuren und/oder deren Derivaten, vorzugsweise Maleinsäureanhydrid, in der Gasphase, **dadurch gekennzeichnet, dass** dieser Katalysator 40-92 Gew.-% Kupferoxid, 0,005-5 Gew.-% Palladium und/oder einer Palladiumverbindung und 2-59,99 Gew.-% eines saure Zentren aufweisenden oxidischen Trägermaterials ausgewählt aus der Gruppe der Oxide des Al, Si, Zn, La, Ce, der Elemente der Gruppen IIIA bis VIIIA sowie der Gruppen IA und IIA aufweist.

2. Katalysator nach Anspruch 1, **dadurch gekennzeichnet, dass** er Palladiumoxid und/oder Palladiumnitrat aufweist.

3. Katalysator nach Anspruch 1, **dadurch gekennzeichnet, dass** das Trägermaterial ausgewählt ist aus der Gruppe bestehend aus Aluminiumoxid, Siliciumoxid, Titanoxid, Zinkoxid, Zirkonoxid und/oder Ceroxid.

4. Katalysator nach einem der Ansprüche 1 bis 3, **dadurch gekennzeichnet, dass** der Katalysator aus Kupferoxid, Palladium und/oder einer Palladiumverbindung und Aluminiumoxid besteht.

5. Verfahren zur Hydrierung von C₄-Dicarbonsäuren und/oder deren Anhydriden oder Estern, vorzugsweise Maleinsäureanhydrid, in der Gasphase, **dadurch gekennzeichnet, dass** die Hydrierung in Gegenwart eines Katalysators nach einem der Ansprüche 1 bis 5 durchgeführt wird.

6. Verfahren nach Anspruch 5, **dadurch gekennzeichnet, daß** die GHSV bei Werten von 10 bis 50 000 h⁻¹, vorzugsweise 100 bis 10.000 h⁻¹, liegt.

7. Verfahren nach Anspruch 5 oder 6, **dadurch gekennzeichnet, daß** die Reaktionstemperatur bei Werten von 150 bis 400°C, vorzugsweise 200 bis 300°C und der Druck während der Reaktion bei Werten von 0,5 bis 50 bar, vorzugsweise 1 bis 20 bar, liegt

8. Verfahren nach einem der Ansprüche 5 bis 7, **dadurch gekennzeichnet, dass** die Konzentration an C₄-Dicarbonsäure oder dessen Derivat bei Werten von 0,5 bis 5 Vol.-%, vorzugsweise 0,2 bis 2 Vol.-%, liegt.

9. Verfahren zur Herstellung eines Katalysators nach einem der Ansprüche 1 bis 4, **dadurch gekennzeichnet, dass** ein mittels an sich bekannter Verfahren hergestellter Katalysator enthaltend Kupferoxid und ein oxidisches Trägermaterial mit einer Lösung einer löslichen Palladiumverbindung versetzt, getrocknet und wahlweise calciniert wird.

## Claims

1. A catalyst for the hydrogenation of C₄-dicarboxylic acids and/or derivatives thereof, preferably maleic anhydride, in the gas phase, which catalyst comprises 40-92% by weight of copper oxide, 0.005-5% by weight of palladium and/or a palladium compound and 2-59.99% by weight of an oxidic support material having acid sites and selected from the group consisting of the oxides of Al, Si, Zn, La, Ce, and the elements of groups IIIA to VIIIA and of groups IA and IIA.

2. A catalyst as claimed in claim 1 which comprises palladium oxide and/or palladium nitrate.

3. A catalyst as claimed in claim 1, wherein the support material is selected from the group consisting of aluminum oxide, silicon oxide, titanium oxide, zinc oxide, zirconium oxide and cerium oxide.

4. A catalyst as claimed in any of claims 1 to 3 which consists of copper oxide, palladium and/or a palladium compound and aluminum oxide.

5. A process for the hydrogenation of C₄-dicarboxylic acids and/or anhydrides or esters thereof, preferably maleic anhydride, in the gas phase in the presence of a catalyst as claimed in any of claims 1 to 5.

6. A process as claimed in claim 5, wherein the GHSV is from 10 to 50 000 h⁻¹, preferably from 100 to 10 000 h⁻¹.

7. A process as claimed in claim 5 or 6, wherein the reaction temperature is from 150 to 400°C, preferably from 200 to 300°C, and the pressure during the reaction is from 0.5 to 50 bar, preferably from 1 to 20 bar.

8. A process as claimed in any of claims 5 to 7, wherein the concentration of C₄-dicarboxylic acid or the derivative thereof is from 0.5 to 5% by volume, preferably from 0.2 to 2% by volume.

9. A process for producing a catalyst as claimed in any of claims 1 to 4, wherein a catalyst comprising copper oxide and an oxidic support material and produced by methods known per se is treated with a solution of a soluble palladium compound, dried and, if desired calcined.

## Revendications

1. Catalyseur pour l'hydrogénation d'acides dicarboxyliques en C₄ et/ou de leurs dérivés, de préférence l'anhydride maléique, en phase gazeuse, **caractérisé en ce que** ce catalyseur présente 40% à 92% en poids d'oxyde de cuivre, 0,005% à 5% en poids de palladium et/ou d'un composé du palladium et 2% à 59,99% en poids d'une matière support contenant un oxyde présentant des centres acides choisis dans le groupe formé par les oxydes d'Al, de Si, de Zn, de La, de Ce, des éléments des groupes IIIA et VIIIA ainsi que des groupes IA et IIA.

2. Catalyseur selon la revendication 1, **caractérisé en ce qu'**il présente de l'oxyde de palladium et/ou du nitrate de palladium.

3. Catalyseur selon la revendication 1, **caractérisé en ce que** la matière support est choisie dans le groupe formé par l'oxyde d'aluminium, l'oxyde de silicium, l'oxyde de titane, l'oxyde de zinc, l'oxyde de zirconium et/ou l'oxyde de cérium.

4. Catalyseur selon l'une quelconque des revendications 1 à 3, **caractérisé en ce que** le catalyseur se compose d'oxyde de cuivre, de palladium et/ou d'un composé du palladium et d'oxyde d'aluminium.

5. Procédé d'hydrogénation d'acides dicarboxyliques en C₄ et/ou de leurs anhydrides ou de leurs esters, par exemple l'anhydride maléique, en phase gazeuse, **caractérisé en ce que** l'on réalise l'hydrogénation en présence d'un catalyseur selon l'une quelconque des revendications 1 à 5.

6. Procédé selon la revendication 5, **caractérisé en ce que** la GHSV se situe à des valeurs allant de 10 h⁻¹ à 50 000 h⁻¹, de préférence de 100 h⁻¹ à 10 000 h⁻¹.

7. Procédé selon la revendication 5 ou 6, **caractérisé en ce que** la température de réaction se situe à des valeurs allant de 150°C à 400°C, de préférence de 200°C à 300°C, et la pression pendant la réaction à des valeurs allant de 0,5 bar à 50 bars, de préférence de 1 bar à 20 bars.

8. Procédé selon l'une quelconque des revendications 1 à 7, **caractérisé en ce que** la concentration d'acide dicarboxylique en C₄ ou de ses dérivés se situe à des valeurs allant de 0,5% à 5% en volume, de préférence de 0,2% à 2% en volume.

9. Procédé de préparation d'un catalyseur selon l'une quelconque des revendications 1 à 4, **caractérisé en ce que** l'on mélange un catalyseur contenant de l'oxyde de cuivre produit au moyen de procédés connus en soi et une matière support contenant un oxyde avec une solution d'un composé soluble du palladium, on le sèche et, éventuellement, on le calcine.
